# EUROPEAN PATENT APPLICATION

(11) **EP 3 835 429 A1**
(43) Date of publication of application: **16.06.2021**
(21) Application number: 20201251.4
(22) Date of filing: 16.10.2015
(51) Int. Cl.: C12Q 1/68, C12Q 1/6869, G16B 20/10

(54) **PRE-IMPLANTATION GENETIC SCREENING AND ANEUPLOIDY DETECTION**

(30) Priority: 17.10.2014 US 201462065322 P
(62) Divisional of application: 15850267.4
(71) Applicant: Good Start Genetics, Inc., Cambridge, MA 02139 (US)
(72) Inventor: PORRECA, Gregory, Cambridge, MA 02138 (US)
(74) Representative: Graham Watt & Co LLP

(57) **Abstract**

Provided herein are methods for determining ploidy of an embryo. The methods can include the steps of amplifying, using a primer pair that amplifies a plurality of human genomic loci, nucleic acid from a preimplantation embryo to generate a plurality of amplicons, sequencing the amplicons to generate a plurality of sequence reads, matching the sequence reads to the genomic loci and counting a number of matches, and determining chromosome count based on the number of matches. Also provided herein are systems for determining chromosome count comprising a processor coupled to a tangible memory subsystem storing instructions. When executed by the processor, the instructions cause the system to implement the methods provided.

## Description

### Cross Reference to Related Applications

This application claims priority to U.S. Provisional Application No. 62/065,322, filed on October 17, 2014, the contents of which are incorporated by reference.

### Field of the Invention

The invention relates to the screening of embryos prior to implantation.

### Background

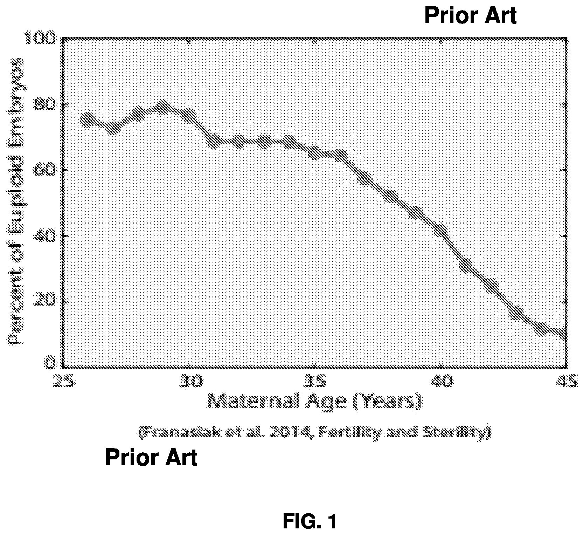

People having difficulty having children may turn to in vitro fertilization (IVF). IVF involves fertilization of an egg outside of the womb followed by implantation of the embryo into the mother. According to the CDC, IVF accounts for 99% of assisted reproductive technology procedures performed in the U.S. However, numerous difficulties with IVF exist. For instance, many of the people turning to IVF are females over the age of 35, the age at which a female is said to be of advanced maternal age and at which the percentage of euploid embryos starts to experience a precipitous drop, as shown in FIG. 1. Accordingly to a 2011 study issued by the CDC, the percentage of IVF cycles resulting in pregnancy in females ages 38-40 is only about 29% and only about 22% resulted in live births. See "2011 Assisted Reproductive Technology: Fertility Clinic Success Rates Report."

A common factor in failed pregnancies is the presence of chromosomal aneuploidies. Aneuploidy is a condition in which the number of chromosomes is not an exact multiple of the haploid number (23 in humans). In contrast, euploidy is the presence of an exact multiple of the haploid number and is considered "normal" in humans. Most aneuploidies are lethal to the fetus, although some, such as trisomy 21 (Down syndrome), trisomy 18 (Edwards syndrome), and trisomy 18 (Patau syndrome), while not always lethal, cause congenital defects, growth deficiencies and intellectual disabilities in the child.

Growing evidence indicates that the chance of achieving a successful pregnancy improves when a euploid embryo(s) is transferred. Pre-implantation genetic screening (PGS) is one method by which the karyotype or chromosome copy number of an embryo or embryos can be assessed such that an aneuploidy or euploidy state can be determined. However, PGS has been limited at least in part due to the high cost associated with traditional PGS approaches and the time it takes to complete the screening.

### Summary

The invention provides systems and methods for improving the success rate of IVF procedures and improving the health and welfare of children conceived through IVF by screening the genetic makeup of candidate embryos for IVF prior to implantation particularly to detect aneuploidy. Pre-implantation genetic screening (PGS) can be used to assess the karyotype or chromosome copy number of embryos, allowing for the determination of a euploidy or aneuploidy state of the embryo. The present invention allows for broader adoption of PGS through the use of procedures, such as trophectoderm biopsy followed by vitrification and subsequent frozen embryo transfer, coupled with streamlined workflows employing next-generation DNA sequencing (NGS), such as FAST-SeqS.

According to one embodiment of the invention, a method is provided for determining ploidy of an embryo. Using a primer pair that amplifies a plurality of human genomic loci, nucleic acid from a preimplantation embryo is amplified to generate a plurality of amplicons. The amplicons are sequenced to generate a plurality of sequence reads. The sequence reads are matched to the genomic loci and a number of matches are counted. The chromosome count is then determined based on the number of matches.

In one aspect of the method, a sample is obtained comprising nucleic acid. In another aspect, the sample is obtained by biopsy. In yet another aspect of the method, the biopsy is a trophectoderm biopsy. In one aspect of the method, the sample includes at least one cell from the preimplantation embryo. In another aspect of the invention, the sample contains from about 1 to about 8 cells. In yet another aspect, the sample contains from about 1 to about 5 cells.

In yet another aspect of the method, the primer pair is complimentary to sequences distributed on at least 4 human chromosomes.

In another aspect of the method, not all of the amplicons are identical. In another aspect, the amplicons include sequences on at least one chromosome of interest and sequences on one or more reference chromosomes. The chromosomes of interest can be include, but is not limited to, chromosome 9, chromosome 13, chromosome 18, chromosome 21, X chromosome and Y chromosome.

In another aspect of the method, chromosome count is determined by the generation and comparison of a z-score for a chromosome of interest.

In yet another aspect of the method, a euploidy or aneuploidy state of the embryo is determined based on the chromosome count.

In another aspect of the method, sequence adapters and bar codes are attached to the amplicons simultaneously with amplification of the nucleic acid. In yet another aspect, the nucleic acid is fragmented.

In another aspect of the method, the primer contains a universal primer binding site. In yet another aspect of the method, a second round of amplification can be done, which includes adding sequencing adaptors to the amplicons using second primers that hybridize to the universal primer binding site.

According to another embodiment of the invention, a system is provided for determining chromosome count. The system includes a processor coupled to a tangible memory subsystem storing instructions. When the instructions are executed by the processor, the system is caused to obtain sequence reads from amplicons, wherein the amplicons are generated by amplifying, using a primer pair that amplifies a plurality of human genomic loci, nucleic acid from a preimplantation embryo. The system then matches the sequence reads to thegenomic loci and counts a number of matches at the genomic loci. Chromosome count is then determined based on the number of matches.

In one aspect of the system, the nucleic acid is obtained from a sample. In another aspect of the system, the sample is obtained by biopsy. In yet another aspect of the system, the biopsy is a trophectoderm biopsy. In another aspect of the system, the sample contains from about 1 to about 5 cells from the preimplantation embryo.

In one aspect of the system, the primer pair is complimentary to sequences distributed on at least 4 human chromosomes. In another aspect, the amplicons include sequences on at least one chromosome of interest and sequences on one or more reference chromosomes. In yet another aspect, the chromosomes of interest are selected from chromosome 9, chromosome 13, chromosome 18, chromosome 21, X chromosome and Y chromosome.

In yet another aspect of the system, the instructions further cause the system to determine and report a euploidy or aneuploidy state of the embryo based on the chromosome count.

### Brief Description of the Drawings

FIG. 1 is a prior art finding relating euploid embryo number to maternal age.
FIG. 2 diagrams methods of certain embodiments of the invention.
FIG. 3 gives an overview of FAST-SeqS based PGS.
FIG. 4 gives an overview of trophectoderm biopsy.
FIG. 5 gives a diagram of a system of the invention.
FIG. 6 shows results from euploid cells.
FIG. 7 shows results from aneuploid cells.
FIG. 8 shows karyotype calls for 2 fibroblast cells diluted.
FIG. 9 shows karyotype calls for 2 fibroblast cells micro-manipulated.
FIG. 10 shows karyotype calls for 5 fibroblast cells diluted.
FIG. 11 shows karyotype calls for 5 fibroblast cells micro-manipulated.
FIG. 12 is a chart summarizing number, specificity, and sensitivity by sample type.

### Detailed Description

Pre-implantation genetic screening (PGS) is the screening of embryos for chromosome abnormalities (e.g., karyotype or aneuploidy testing) prior to implantation in an in vitro fertilization setting . By conducting PGS, the potential of transferring an embryo(s) with the correct number of chromosomes increases as does the potential for increased pregnancy rates.

Most cells in the human body have 23 pairs of chromosomes, or a total of 46 chromosomes. One copy of each pair is inherited from the mother and the other copy is inherited from the father. The first 22 pairs of chromosomes (called autosomes) are numbered from 1 to 22, from largest to smallest. The 23rd pair of chromosomes are the sex chromosomes. Normal females have two X chromosomes, while normal males have one X chromosome and one Y chromosome. Disomy is the presence of two copies of a chromosome. For organisms such as humans, two copies of each chromosome (i.e., diploid) is the normal condition.

During meiosis, when germ cells divide to create sperm and egg (gametes), each half should have the same number of chromosomes. But sometimes, the whole pair of chromosomes will end up in one gamete, and the other gamete will not get that chromosome at all. The presence of an abnormal number of chromosomes in a cell is referred to as aneuploidy. An extra or missing chromosome is a common cause of genetic disorders, including some human birth defects. Types of aneuploidy include monosomy (one copy of a chromosome), trisomy (three copies of a chromosome), and tetrasomy (four copies of a chromosome). The key objective of PGS is to accurately determine the copy number of each chromosome. By accurately calling the chromosome copy number, it is possible to identify aneuploidy.

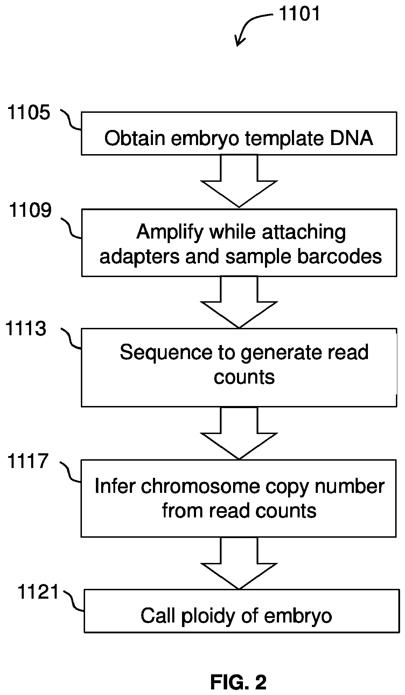

Figure 2 diagrams a general method 1101 according to certain embodiments of the invention. As shown, embryo template DNA is obtained 1105 from a sample. The DNA is amplified to provide amplicons, while adapters and sample barcodes are simultaneously attached 1109. The amplicons are then sequenced to generate read counts 1113. The read counts can be used to infer chromosome copy number 1117. Based on the copy number/read counts, the ploidy of the embryo can be determined, or "called" 1121.

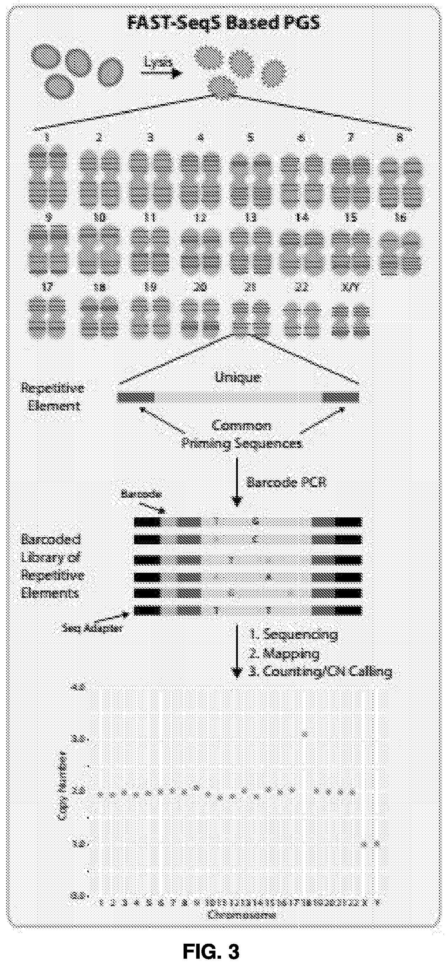

Figure 3 provides an overview of one embodiment of the invention using FAST-SeqS based PGS. Cells are obtained and lysed to release nucleic acid from 23 chromosomes. The fragments are amplified using a single primer pair designed to amplify a discrete subset of repeated regions to provide amplicons. Sequence adapters and bar codes can be attached to the amplicons simultaneously with the amplification of the nucleic acid. The amplicons are then sequenced and matched to sequences at genomic loci. The number of matches are counted to determine the copy number, or "call" the copy number.

In order to obtain a viable embryo(s) for implantation, a typical procedure is for the female patient to undergo controlled ovarian stimulation (COH) to produce a large group of oocytes (e.g., developing eggs). The oocytes are retrieved and denudated from the cumulus cells, as these cells can be a source of contamination during analysis. IVF can be used to fertilize the oocyte. One example of an IVF procedure used to fertilize the oocyte is intracytoplasmic sperm injection (ICSI). ICSI involves the injection of a single sperm directly into an egg. Once fertilized, embryo development is typically evaluated every day prior to biopsy for PGS purposes.

There are several biopsy methods by which nucleic acid can be obtained from a sample to carryout PGS. The methods differ depending on the preimplantation stage at which the biopsy will be performed. Exemplary biopsy methods include but are not limited to polar body biopsy, cleavage-stage biopsy (blastomere biopsy), and blastocyst biopsy (trophectoderm biopsy).

A polar body (PB) biospy is the sampling of a polar body, which is a small haploid cell that is formed concomitantly as an egg cell during oogenesis, but which generally does not have the ability to be fertilized. The main advantage of the use of polar bodies in PGS is that they are not necessary for successful fertilization or normal embryonic development, thus ensuring no deleterious effect for the embryo. One of the disadvantages of PB biopsy is that it only provides information about the maternal contribution to the embryo, which is why cases of autosomal dominant and X-linked disorders that are maternally transmitted can be diagnosed, and autosomal recessive disorders can only partially be diagnosed. See "Delivery of a chromosomally normal child from an oocyte with reciprocal aneuploid polar bodies". Scott Jr, Richard T., Nathan R. Treff, John Stevens, Eric J. Forman, Kathleen H. Hong, Mandy G. Katz-Jaffe, William B. Schoolcraft. Journal of Assisted Reproductive Genetics Vol. 29 pp. 533-537. 2012.

Cleavage-stage biopsy is generally performed the morning of day three post-fertilization, when normally developing embryos reach the eight-cell stage. A hole is made in the zona pellucida and one or more blastomeres containing a nucleus are gently aspirated or extruded through the opening. One of the advantages of cleavage-stage biopsy is that the genetic input of both parents can be studied. One of the disadvantages is that cleavage-stage embryos are found to have a high rate of chromosomal mosaicism, i.e., the presence of two or more populations of cells with different genotypes in one individual. Because of this, it is possible that the results obtained on the blastomeres will not be representative for the rest of the embryo.

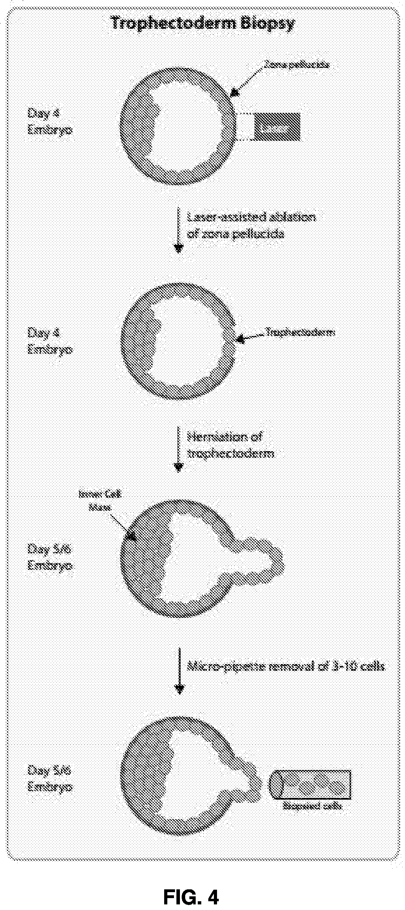

Trophectoderm biopsy involves removing cells from the trophectoderm component of an IVF blastocyst embryo. Trophectoderm is the outer layer of the mammalian blastocyst after differentiation of the ectoderm, mesoderm, and endoderm when the outer layer is continuous with the ectoderm of the embryo. As shown in FIG. 4, the process involves making a hole in the zona pellucida on day three of in vitro culture. The trophectoderm will then protrude after blastulation, facilitating the biopsy. On day five post-fertilization, typically about five cells are excised from the trophectoderm using a glass needle or laser energy, leaving the embryo largely intact and without loss of inner cell mass. However, it is to be understood that the number of cells excised can be from about 1 to about 8 cells, or from about 1 to about 5 cell, or about 5 cells. It is also to be understood that more or less than 5, such as, for example but not limitation, 1, 2, 3, 4, 6, 7 or 8 cells can be excised. The removed cells can then be tested for overall chromosome normality. After diagnosis, depending on the amount of time it takes to obtain the results from PGS, the embryos can be replaced during the same cycle, or cryopreserved and transferred in a subsequent cycle. Oocyte cryopreservation (e.g., "egg freezing") refers to the process in which a woman's oocytes (eggs) are extracted, frozen and stored. One type of cryopreservation process that has become increasingly popular is vitfication. Vitrification is an ultra-rapid cryopreservation process that involves the use of high concentrations of cryoprotectants.

Once a sample is obtained, nucleic acid is isolated from the sample for analysis. Generally, nucleic acid can be extracted from a biological sample by a variety of techniques such as those described by Maniatis, et al., Molecular Cloning: A Laboratory Manual, 1982, Cold Spring Harbor, N.Y., pp. 280-281; Sambrook and Russell, Molecular Cloning: A Laboratory Manual 3Ed, Cold Spring Harbor Laboratory Press, 2001, Cold Spring Harbor, N.Y.; or as described in U.S. Pub. 2002/0190663.

Nucleic acid obtained from biological samples can be fragmented to produce suitable fragments for analysis. Template nucleic acids may be fragmented or sheared to desired length, using a variety of mechanical, chemical and/or enzymatic methods. DNA may be randomly sheared via sonication, e.g. Covaris method, brief exposure to a DNase, or using a mixture of one or more restriction enzymes, or a transposase or nicking enzyme. RNA may be fragmented by brief exposure to an RNase, heat plus magnesium, or by shearing. The RNA may be converted to cDNA. If fragmentation is employed, the RNA may be converted to cDNA before or after fragmentation. In one embodiment, nucleic acid from a biological sample is fragmented by sonication. In another embodiment, nucleic acid is fragmented by a hydroshear instrument. Generally, individual nucleic acid template molecules can be from about 2 kb bases to about 40 kb. In a particular embodiment, nucleic acids are about 6 kb-10 kb fragments. Nucleic acid molecules may be single-stranded, double-stranded, or double-stranded with single-stranded regions (for example, stem- and loop-structures).

A biological sample as described herein may be homogenized or fractionated in the presence of a detergent or surfactant. The concentration of the detergent in the buffer may be about 0.05% to about 10.0%. The concentration of the detergent can be up to an amount where the detergent remains soluble in the solution. In one embodiment, the concentration of the detergent is between 0.1% to about 2%. The detergent, particularly a mild one that is nondenaturing, can act to solubilize the sample. Detergents may be ionic or nonionic. Examples of nonionic detergents include triton, such as the Triton® X series (Triton® X-100 t-Oct-C₆H₄-(OCH₂-CH₂)ₓOH, x=9-10, Triton® X-100R, Triton® X-114 x=7-8), octyl glucoside, polyoxyethylene(9)dodecyl ether, digitonin, IGEPAL® CA630 octylphenyl polyethylene glycol, n-octyl-beta-D-glucopyranoside (betaOG), n-dodecyl-beta, Tween® 20 polyethylene glycol sorbitan monolaurate, Tween® 80 polyethylene glycol sorbitan monooleate, polidocanol, n-dodecyl beta-D-maltoside (DDM), NP-40 nonylphenyl polyethylene glycol, C12E8 (octaethylene glycol n-dodecyl monoether), hexaethyleneglycol mono-n-tetradecyl ether (C 14EO6), octyl-beta-thioglucopyranoside (octyl thioglucoside, OTG), Emulgen, and polyoxyethylene 10 lauryl ether (C12E10). Examples of ionic detergents (anionic or cationic) include deoxycholate, sodium dodecyl sulfate (SDS), N-lauroylsarcosine, and cetyltrimethylammoniumbromide (CTAB). A zwitterionic reagent may also be used in the purification schemes of the present invention, such as Chaps, zwitterion 3-14, and 3-[(3-cholamidopropyl)dimethyl-ammonio]-1-propanesulfonate. It is contemplated also that urea may be added with or without another detergent or surfactant.

Lysis or homogenization solutions may further contain other agents, such as reducing agents. Examples of such reducing agents include dithiothreitol (DTT), β-mercaptoethanol, DTE, GSH, cysteine, cysteamine, tricarboxyethyl phosphine (TCEP), or salts of sulfurous acid.

In various embodiments, the nucleic acid is amplified, for example, from the sample or after isolation from the sample. In one embodiment, the nucleic acid is amplified after isolation and fragmentation to provide amplicons. In another embodiment, the nucleic acid is amplified without the need for fragmentation. Amplification refers to production of additional copies of a nucleic acid sequence and is generally carried out using primers in polymerase chain reaction or other technologies well known in the art (e.g., Dieffenbach and Dveksler, PCR Primer, a Laboratory Manual, 1995, Cold Spring Harbor Press, Plainview, N.Y.). The amplification reaction may be any amplification reaction known in the art that amplifies nucleic acid molecules, such as polymerase chain reaction (PCR), nested polymerase chain reaction, polymerase chain reaction-single strand conformation polymorphism, ligase chain reaction (Barany, F., Genome Research, 1:5-16 (1991); Barany, F., PNAS, 88:189-193 (1991); U.S. Pat. No. 5,869,252; and U.S. Pat. No. 6,100,099), strand displacement amplification and restriction fragments length polymorphism, transcription based amplification system, rolling circle amplification, and hyper-branched rolling circle amplification. Further examples of amplification techniques that can be used include, but are not limited to, quantitative PCR, quantitative fluorescent PCR (QF-PCR), multiplex fluorescent PCR (MF-PCR), real time PCR (RTPCR), single cell PCR, restriction fragment length polymorphism PCR (PCR-RFLP), RT-PCR-RFLP, hot start PCR, in situ polonony PCR, in situ rolling circle amplification (RCA), bridge PCR, picotiter PCR and emulsion PCR. Other suitable amplification methods include transcription amplification, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP-PCR) and nucleic acid based sequence amplification (NABSA). Other amplification methods that can be used herein include those described in U.S. Pat. Nos. 5,242,794; 5,494,810; 4,988,617; and 6,582,938.

In certain embodiments, the amplification reaction can include polymerase chain reaction (PCR). PCR refers to methods by K. B. Mullis (U.S. Pat. Nos. 4,683,195 and 4,683,202, hereby incorporated by reference) for increasing concentration of a segment of a target sequence in a mixture of genomic DNA without cloning or purification.

In one embodiment, the amplification method can include the method described in Kinde et al., 2012, FAST-SeqS: a simple and efficient method for the detection of aneuploidy by massively parallel sequencing, PLoS One 7(7):e41162, wherein a single primer pair is used to produce amplicons. By using the FAST-SeqS ("Fast Aneuploidy Screening Test-Sequencing"), the need for end-repair, terminal 3'dA addition, or ligation to adapters can be obviated.

Primers can be prepared by a variety of methods including but not limited to cloning of appropriate sequences and direct chemical synthesis using methods well known in the art (Narang et al., Methods Enzymol., 68:90 (1979); Brown et al., Methods Enzymol., 68:109 (1979)). Primers can also be obtained from commercial sources such as Operon Technologies, Amersham Pharmacia Biotech, Sigma, and Life Technologies. The primers can have an identical melting temperature. The lengths of the primers can be extended or shortened at the 5' end or the 3' end to produce primers with desired melting temperatures. Also, the annealing position of each primer pair can be designed such that the sequence and length of the primer pairs yield the desired melting temperature. The simplest equation for determining the melting temperature of primers smaller than 25 base pairs is the Wallace Rule (Td=2(A+T)+4(G+C)). Computer programs can also be used to design primers, including but not limited to Array Designer Software from Arrayit Corporation (Sunnyvale, Calif.), Oligonucleotide Probe Sequence Design Software for Genetic Analysis from Olympus Optical Co., Ltd. (Tokyo, Japan), NetPrimer, and DNAsis Max v3.0 from Hitachi Solutions America, Ltd. (South San Francisco, Calif.). The TM (melting or annealing temperature) of each primer is calculated using software programs such as OligoAnalyzer 3.1, available on the web site of Integrated DNA Technologies, Inc. (Coralville, Iowa).

In one embodiment, the primer is a single primer pair that can anneal to a subset of human sequences dispersed throughout the genome. See Kinde et al., 2012, incorporated herein. Preferably, the primer is a single primer pair that can amplify many distinct fragments of nucleic acid from throughout the genome as well as throughout the critical region(s) of the chromosome or chromosomes of interest to produce amplicons. In a preferred embodiment, not all of the amplicons are identical. The primer pairs can be complementary to sequences on at least 3, 4, 5, 6, 7, 8, 9, 10, 11, 12, 13, 14, 15, 16, 17, 18, 19, 20, 21, 22 or 23 human chromosomes. As such, it is possible for the amplicons to include sequences on one or more reference chromosomes and at least one chromosome of interest. In one embodiment, the chromosomes of interest include chromosome 9, chromosome 13, chromosome 18, chromosome 21, X chromosome and Y chromosome.

Amplification adapters can be attached to the fragmented nucleic acid. Adapters may be commercially obtained, such as from Integrated DNA Technologies (Coralville, Iowa). In certain embodiments, the adapter sequences are attached to the template nucleic acid molecule with an enzyme. The enzyme may be a ligase or a polymerase. The ligase may be any enzyme capable of ligating an oligonucleotide (RNA or DNA) to the template nucleic acid molecule. Suitable ligases include T4 DNA ligase and T4 RNA ligase, available commercially from New England Biolabs (Ipswich, Mass.). Methods for using ligases are well known in the art. The polymerase may be any enzyme capable of adding nucleotides to the 3' and the 5' terminus of template nucleic acid molecules.

Additionally, the primer can comprise a universal primer bonding site, such that if a second round of amplification is completed, sequence adapters can be added to the amplicons using second primers that hybridize to the universal primer binding site.

In certain embodiments, bar codes, or tags, can be attached to one or more fragments or amplicons. For example, but not limitation, the barcodes can be attached to a plurality of fragments or amplicons, or each of the fragments or amplicons. In one embodiment, a single bar code can be attached to a fragment or amplicon. In other embodiments, a plurality of bar codes, e.g., two or more bar codes, can be attached to a fragment or amplicon.

A bar code sequence generally includes certain features that make the sequence useful in sequencing reactions. For example the bar code sequences can be designed to have minimal or no homopolymer regions, i.e., 2 or more of the same base in a row such as AA or CCC, within the bar code sequence. The bar code sequences can also be designed so that they are at least one edit distance away from the base addition order when performing base-by-base sequencing, ensuring that the first and last base do not match the expected bases of the sequence.

The bar code sequences can also be designed such that each sequence is correlated to a particular portion of nucleic acid, allowing sequence reads to be correlated back to the portion from which they came. Methods of designing sets of bar code sequences is shown for example in U.S. Pat. No. 6,235,475, the contents of which are incorporated by reference herein in their entirety. In certain embodiments, the bar code sequences can range from about 5 nucleotides to about 15 nucleotides. In a particular embodiment, the bar code sequences can range from about 4 nucleotides to about 7 nucleotides. Since the bar code sequence is sequenced along with the template nucleic acid, the oligonucleotide length should be of minimal length so as to permit the longest read from the template nucleic acid attached. Generally, the bar code sequences can be spaced from the template nucleic acid molecule by at least one base (minimizes homopolymeric combinations).

Methods of the invention involve attaching the bar code sequences to the template nucleic acids. In certain embodiments, the bar code sequences are attached to the template nucleic acid molecule with an enzyme. The enzyme may be a ligase or a polymerase, as discussed above. Attaching bar code sequences to nucleic acid templates is shown in U.S. Pub. 2008/0081330 and U.S. Pub. 2011/0301042, the content of each of which is incorporated by reference herein in its entirety. Methods for designing sets of bar code sequences and other methods for attaching bar code sequences are shown in U.S. Pat. Nos. 6,138,077; 6,352,828; 5,636,400; 6,172,214; 6235,475; 7,393,665; 7,544,473; 5,846,719; 5,695,934; 5,604,097; 6,150,516; RE39,793; 7,537,897; 6,172,218; and 5,863,722, the content of each of which is incorporated by reference herein in its entirety. In one embodiment, sequence adapters and sample-specific barcodes can be simultaneously attached as regions from each chromosome are amplified.

After any processing steps (e.g., obtaining, isolating, fragmenting, or amplification), nucleic acid can be sequenced according to certain embodiments of the invention. Sequencing may be by any method known in the art. DNA sequencing techniques include classic dideoxy sequencing reactions (Sanger method) using labeled terminators or primers and gel separation in slab or capillary, sequencing by synthesis using reversibly terminated labeled nucleotides, pyrosequencing, 454 sequencing, Illumina/Solexa sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, polony sequencing, and SOLiD sequencing. Sequencing of separated molecules has more recently been demonstrated by sequential or single extension reactions using polymerases or ligases as well as by single or sequential differential hybridizations with libraries of probes.

A sequencing technique that can be used in the methods of the provided invention includes, for example, 454 sequencing (454 Life Sciences, a Roche company, Branford, Conn.) (Margulies, M et al., Nature, 437:376-380 (2005); U.S. Pat. No. 5,583,024; U.S. Pat. No. 5,674,713; and U.S. Pat. No. 5,700,673). 454 sequencing involves two steps. In the first step, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is detected and analyzed.

Another example of a DNA sequencing technique that can be used in the methods of the provided invention is SOLiD technology by Applied Biosystems from Life Technologies Corporation (Carlsbad, Calif.). In SOLiD sequencing, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

Another example of a DNA sequencing technique that can be used in the methods of the provided invention is Ion Torrent sequencing, described, for example, in U.S. Pubs. 2009/0026082, 2009/0127589, 2010/0035252, 2010/0137143, 2010/0188073, 2010/0197507, 2010/0282617, 2010/0300559, 2010/0300895, 2010/0301398, and 2010/0304982, the content of each of which is incorporated by reference herein in its entirety. In Ion Torrent sequencing, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to a surface and are attached at a resolution such that the fragments are individually resolvable. Addition of one or more nucleotides releases a proton (H⁺), which signal is detected and recorded in a sequencing instrument. The signal strength is proportional to the number of nucleotides incorporated.

Another example of a sequencing technology that can be used in the methods of the provided invention is Illumina sequencing. Illumina sequencing is based on the amplification of DNA on a solid surface using fold-back PCR and anchored primers. Genomic DNA is fragmented, and adapters are added to the 5' and 3' ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated. Sequencing according to this technology is described in U.S. Pub. 2011/0009278, U.S. Pub. 2007/0114362, U.S. Pub. 2006/0024681, U.S. Pub. 2006/0292611, U.S. Pat. No. 7,960,120, U.S. Pat. No. 7,835,871, U.S. Pat. No. 7,232,656, U.S. Pat. No. 7,598,035, U.S. Pat. No. 6,306,597, U.S. Pat. No. 6,210,891, U.S. Pat. No. 6,828,100, U.S. Pat. No. 6,833,246, and U.S. Pat. No. 6,911,345, each of which are herein incorporated by reference in their entirety.

Another example of a sequencing technology that can be used in the methods of the provided invention includes the single molecule, real-time (SMRT) technology of Pacific Biosciences (Menlo Park, Calif.). In SMRT, each of the four DNA bases is attached to one of four different fluorescent dyes. These dyes are phospholinked. A single DNA polymerase is immobilized with a single molecule of template single stranded DNA at the bottom of a zero-mode waveguide (ZMW). A ZMW is a confinement structure which enables observation of incorporation of a single nucleotide by DNA polymerase against the background of fluorescent nucleotides that rapidly diffuse in and out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated.

Another example of a sequencing technique that can be used in the methods of the provided invention is nanopore sequencing (Soni, G. V., and Meller, A., Clin Chem 53: 1996-2001 (2007)). A nanopore is a small hole, of the order of 1 nanometer in diameter. Immersion of a nanopore in a conducting fluid and application of a potential across it results in a slight electrical current due to conduction of ions through the nanopore. The amount of current which flows is sensitive to the size of the nanopore. As a DNA molecule passes through a nanopore, each nucleotide on the DNA molecule obstructs the nanopore to a different degree. Thus, the change in the current passing through the nanopore as the DNA molecule passes through the nanopore represents a reading of the DNA sequence.

Another example of a sequencing technique that can be used in the methods of the provided invention involves using a chemical-sensitive field effect transistor (chemFET) array to sequence DNA (for example, as described in U.S. Pub. 2009/0026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be detected by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

Another example of a sequencing technique that can be used in the methods of the provided invention involves using an electron microscope (Moudrianakis E. N. and Beer M., PNAS, 53:564-71(1965)). In one example of the technique, individual DNA molecules are labeled using metallic labels that are distinguishable using an electron microscope. These molecules are then stretched on a flat surface and imaged using an electron microscope to measure sequences.

Another example of a sequencing technique that can be used in the methods of the provided invention involves the use of FAST-SeqS technology. See FAST-SeqS uses PCR employing a single primer pair that is designed to amplify a discrete subset of repeated regions. In this way, the sequencing process is streamlined, due to the fact that steps such as end-repair, terminal 3 '-dA addition, or ligation to adapters are no longer needed. Furthermore, the smaller number of fragments to be assessed (compared to the whole genome) streamlines the genome matching and analysis processes.

Sequencing according to embodiments of the invention generates a plurality of reads. Reads according to the invention generally include sequences of nucleotide data of less than 500 bases in length, less than 200 bases, or less than, for example, about 175 bases. In one embodiment, the reads are about 150 bases in length.

Following sequencing, reads can be mapped to a reference using assembly and alignment techniques known in the art or developed for use. Various strategies for the alignment and assembly of sequence reads, including the assembly of sequence reads into contigs, are described in detail in U.S. Pat. 8,209,130, incorporated herein by reference. Strategies may include (i) assembling reads into contigs and aligning the contigs to a reference; (ii) aligning individual reads to the reference; (iii) assembling reads into contigs, aligning the contigs to a reference, and aligning the individual reads to the contigs; or (iv) other strategies known to be developed or known in the art. Mapping may employ assembly steps, alignment steps, or both. Assembly can be implemented by the use of any of one programs available in the art. For example, but not limitation, mapping can be done by the program 'The Short Sequence Assembly by k-mer search and 3' read Extension ' (SSAKE), from Canada's Michael Smith Genome Sciences Centre (Vancouver, B.C., CA) (see, e.g., Warren et al., 2007, Assembling millions of short DNA sequences using SSAKE, Bioinformatics, 23:500-501). SSAKE cycles through a table of reads and searches a prefix tree for the longest possible overlap between any two sequences. SSAKE clusters reads into contigs.

A contig, generally, refers to the relationship between or among a plurality of segments of nucleic acid sequences, e.g., reads. Where sequence reads overlap, a contig can be represented as a layered image of overlapping reads. A contig is not defined by, nor limited to, any particular visual arrangement nor any particular arrangement within, for example, a text file or a database. A contig generally includes sequence data from a number of reads organized to correspond to a portion of a sequenced nucleic acid. A contig can include assembly results-such as a set of reads or information about their positions relative to each other or to a reference-displayed or stored. A contig can be structured as a grid, in which rows are individual sequence reads and columns include the base of each read that is presumed to align to that site. A consensus sequence can be made by identifying the predominant base in each column of the assembly. A contig according to the invention can include the visual display of reads showing them overlap (or not, e.g., simply abutting) one another. A contig can include a set of coordinates associated with a plurality of reads and giving the position of the reads relative to each other. A contig can include data obtained by transforming the sequence data of reads. For example, a Burrows-Wheeler transformation can be performed on the reads, and a contig can include the transformed data without necessarily including the untransformed sequences of the reads. A Burrows-Wheeler transform of nucleotide sequence data is described in U.S. Pub. 2005/0032095, herein incorporated by reference in its entirety.

Reads can be assembled into contigs by any method known in the art. Algorithms for the de novo assembly of a plurality of sequence reads are known in the art. One algorithm for assembling sequence reads is known as overlap consensus assembly. Overlap consensus assembly uses the overlap between sequence reads to create a link between them. The reads are generally linked by regions that overlap enough that non-random overlap is assumed Linking together reads in this way produces a contig or an overlap graph in which each node corresponds to a read and an edge represents an overlap between two reads. Assembly with overlap graphs is described, for example, in U.S. Pat. No. 6,714,874.

In some embodiments, de novo assembly proceeds according to so-called greedy algorithms. For assembly according to greedy algorithms, one of the reads of a group of reads is selected, and it is paired with another read with which it exhibits a substantial amount of overlap-generally it is paired with the read with which it exhibits the most overlap of all of the other reads. Those two reads are merged to form a new read sequence, which is then put back in the group of reads and the process is repeated. Assembly according to a greedy algorithm is described, for example, in Schatz, et al., Genome Res., 20:1165-1173 (2010) and U.S. Pub. 2011/0257889, each of which is hereby incorporated by reference in its entirety.

In other embodiments, assembly proceeds by pairwise alignment, for example, exhaustive or heuristic (e.g., not exhaustive) pairwise alignment. Alignment, generally, is discussed in more detail below. Exhaustive pairwise alignment, sometimes called a "brute force" approach, calculates an alignment score for every possible alignment between every possible pair of sequences among a set. Assembly by heuristic multiple sequence alignment ignores certain mathematically unlikely combinations and can be computationally faster. One heuristic method of assembly by multiple sequence alignment is the so-called "divide-and-conquer" heuristic, which is described, for example, in U.S. Pub. 2003/0224384. Another heuristic method of assembly by multiple sequence alignment is progressive alignment, as implemented by the program ClustalW (see, e.g., Thompson, et al., Nucl. Acids. Res., 22:4673-80 (1994)). Assembly by multiple sequence alignment in general is discussed in Lecompte, O., et al., Gene 270:17-30 (2001); Mullan, L. J., Brief Bioinform., 3:303-5 (2002); Nicholas, H. B. Jr., et al., Biotechniques 32:572-91(2002); and Xiong, G., Essential Bioinformatics, 2006, Cambridge University Press, New York, N.Y.

Assembly by alignment can proceed by aligning reads to each other or by aligning reads to a reference. For example, by aligning each read, in turn, to a reference genome, all of the reads are positioned in relationship to each other to create the assembly.

One method of assembling reads into contigs involves making a de Bruijn graph. De Bruijn graphs reduce the computation effort by breaking reads into smaller sequences of DNA, called k-mers, where the parameter k denotes the length in bases of these sequences. In a de Bruijn graph, all reads are broken into k-mers (all subsequences of length k within the reads) and a path between the k-mers is calculated. In assembly according to this method, the reads are represented as a path through the k-mers. The de Bruijn graph captures overlaps of length k-1 between these k-mers and not between the actual reads. Thus, for example, the sequencing CATGGA could be represented as a path through the following 2-mers: CA, AT, TG, GG, and GA. The de Bruijn graph approach handles redundancy well and makes the computation of complex paths tractable. By reducing the entire data set down to k-mer overlaps, the de Bruijn graph reduces the high redundancy in short-read data sets. The maximum efficient k-mer size for a particular assembly is determined by the read length as well as the error rate. The value of the parameter k has significant influence on the quality of the assembly. Estimates of good values can be made before the assembly, or the optimal value can be found by testing a small range of values. Assembly of reads using de Bruijn graphs is described in U.S. Pub. 2011/0004413, U.S. Pub. 2011/0015863, and U.S. Pub. 2010/0063742, each of which are herein incorporated by reference in their entirety.

Other methods of assembling reads into contigs according to the invention are possible. For example, the reads may contain barcode information inserted into template nucleic acid during sequencing. In certain embodiments, reads are assembled into contigs by reference to the barcode information. For example, the barcodes can be identified and the reads can be assembled by positioning the barcodes together.

In certain embodiments, assembly proceeds by making reference to supplied information about the expected position of the various reads relative to each other. This can be obtained, for example, if the subject nucleic acid being sequenced has been captured by molecular inversion probes, because the start of each read derives from a genomic position that is known and specified by the probe set design. Each read can be collected according to the probe from which it was designed and positioned according to its known relative offset. In some embodiments, information about the expected position of reads relative to each other is supplied by knowledge of the positions (e.g., within a gene) of an area of nucleic acid amplified by primers. For example, sequencing can be done on amplification product after a number of regions of the target nucleic acid are amplified using primer pairs designed or known to cover those regions. Reads can then be positioned during assembly at least based on which primer pair was used in an amplification that lead to those reads. Assembly of reads into contigs can proceed by any combination or hybrid of methods including, but not limited to, the above-referenced methods.

Assembly of reads into contigs is further discussed in Husemann, P. and Stoye, J, Phylogenetic Comparative Assembly, 2009, Algorithms in Bioinformatics: 9th International Workshop, pp. 145-156, Salzberg, S., and Warnow, T., Eds. Springer-Verlag, Berlin Heidelberg. Some exemplary methods for assembling reads into contigs are described, for example, in U.S. Pat. No. 6,223,128, U.S. Pub. 2009/0298064, U.S. Pub. 2010/0069263, and U.S. Pub. 2011/0257889, each of which is incorporated by reference herein in its entirety.

Computer programs for assembling reads are known in the art. Such assembly programs can run on a single general-purpose computer, on a cluster or network of computers, or on a specialized computing devices dedicated to sequence analysis.

Assembly can be implemented, for example, by the program 'The Short Sequence Assembly by k-mer search and 3' read Extension' (SSAKE), from Canada's Michael Smith Genome Sciences Centre (Vancouver, B.C., CA) (see, e.g., Warren, R., et al., Bioinformatics, 23:500-501 (2007)). SSAKE cycles through a table of reads and searches a prefix tree for the longest possible overlap between any two sequences. SSAKE clusters reads into contigs.

Another read assembly program is Forge Genome Assembler, written by Darren Platt and Dirk Evers and available through the SourceForge web site maintained by Geeknet (Fairfax, Va.) (see, e.g., DiGuistini, S., et al., Genome Biology, 10:R94 (2009)). Forge distributes its computational and memory consumption to multiple nodes, if available, and has therefore the potential to assemble large sets of reads. Forge was written in C++ using the parallel MPI library. Forge can handle mixtures of reads, e.g., Sanger, 454, and Illumina reads.

Assembly through multiple sequence alignment can be performed, for example, by the program Clustal Omega, (Sievers F., et al., Mol Syst Biol 7 (2011)), ClustalW, or ClustalX (Larkin M. A., et al., Bioinformatics, 23, 2947-2948 (2007)) available from University College Dublin (Dublin, Ireland).

Another exemplary read assembly program known in the art is Velvet, available through the web site of the European Bioinformatics Institute (Hinxton, UK) (Zerbino D. R. et al., Genome Research 18(5):821-829 (2008)). Velvet implements an approach based on de Bruijn graphs, uses information from read pairs, and implements various error correction steps.

Read assembly can be performed with the programs from the package SOAP, available through the website of Beijing Genomics Institute (Beijing, CN) or BGI Americas Corporation (Cambridge, Mass.). For example, the SOAPdenovo program implements a de Bruijn graph approach. SOAP3/GPU aligns short reads to a reference sequence.

Another read assembly program is ABySS, from Canada's Michael Smith Genome Sciences Centre (Vancouver, B.C., CA) (Simpson, J. T., et al., Genome Res., 19(6):1117-23 (2009)). ABySS uses the de Bruijn graph approach and runs in a parallel environment.

Read assembly can also be done by Roche's GS De Novo Assembler, known as gsAssembler or Newbler (NEW assemBLER), which is designed to assemble reads from the Roche 454 sequencer (described, e.g., in Kumar, S. et al., Genomics 11:571(2010) and Margulies, et al., Nature 437:376-380 (2005)). Newbler accepts 454 Fix Standard reads and 454 Titanium reads as well as single and paired-end reads and optionally Sanger reads. Newbler is run on Linux, in either 32 bit or 64 bit versions. Newbler can be accessed via a command-line or a Java-based GUI interface.

Cortex, created by Mario Caccamo and Zamin Iqbal at the University of Oxford, is a software framework for genome analysis, including read assembly. Cortex includes cortex_con for consensus genome assembly, used as described in Spanu, P. D., et al., Science 330(6010):1543-46 (2010). Cortex includes cortex_var for variation and population assembly, described in Iqbal, et al., De novo assembly and genotyping of variants using colored de Bruijn graphs, Nature Genetics (in press), and used as described in Mills, R. E., et al., Nature 470:59-65 (2010). Cortex is available through the creators' web site and from the SourceForge web site maintained by Geeknet (Fairfax, Va.).

Other read assembly programs include RTG Investigator from Real Time Genomics, Inc. (San Francisco, Calif.); iAssembler (Zheng, et al., BMC Bioinformatics 12:453 (2011)); TgiCL Assembler (Pertea, et al., Bioinformatics 19(5):651-52 (2003)); Maq (Mapping and Assembly with Qualities) by Heng Li, available for download through the SourceForge website maintained by Geeknet (Fairfax, Va.); MIRA3 (Mimicking Intelligent Read Assembly), described in Chevreux, B., et al., Genome Sequence Assembly Using Trace Signals and Additional Sequence Information, 1999, Computer Science and Biology: Proceedings of the German Conference on Bioinformatics (GCB) 99:45-56; PGA4genomics (described in Zhao F., et al., Genomics. 94(4):284-6 (2009)); and Phrap (described, e.g., in de la Bastide, M. and McCombie, W. R., Current Protocols in Bioinformatics, 17:11.4.1-11.4.15 (2007)). CLC cell is a de Bruijn graph-based computer program for read mapping and de novo assembly of NGS reads available from CLC bio Germany (Muehltal, Germany).

Once the reads have been assembled into contigs, the contig can be positioned along a reference genome. In certain embodiments, a contig is positioned on a reference through information from known molecular markers or probes. In some embodiments, protein-coding sequence data in a contig or reference genome is represented by amino acid sequence and a contig is positioned along a reference genome. In some embodiments, a contig is positioned by an alignment of the contig to a reference genome.

Alignment, as used herein, generally involves placing one sequence along another sequence, iteratively introducing gaps along each sequence, scoring how well the two sequences match, and preferably repeating for various positions along the reference. The best-scoring match is deemed to be the alignment and represents an inference about the historical relationship between the sequences. In an alignment, a base in the read alongside a non-matching base in the reference indicates that a substitution mutation has occurred at that point. Similarly, where one sequence includes a gap alongside a base in the other sequence, an insertion or deletion mutation (an "indel") is inferred to have occurred. When it is desired to specify that one sequence is being aligned to one other, the alignment is sometimes called a pairwise alignment. Multiple sequence alignment generally refers to the alignment of two or more sequences, including, for example, by a series of pairwise alignments.

In some embodiments, scoring an alignment involves setting values for the probabilities of substitutions and indels. When individual bases are aligned, a match or mismatch contributes to the alignment score by a substitution probability, which could be, for example, 1 for a match and 0.33 for a mismatch. An indel deducts from an alignment score by a gap penalty, which could be, for example, -1. Gap penalties and substitution probabilities can be based on empirical knowledge or a priori assumptions about how sequences mutate. Their values affects the resulting alignment. Particularly, the relationship between the gap penalties and substitution probabilities influences whether substitutions or indels will be favored in the resulting alignment.

Stated formally, an alignment represents an inferred relationship between two sequences, x and y. For example, in some embodiments, an alignment A of sequences x and y maps x and y respectively to another two strings x' and y' that may contain spaces such that: (i) |x'|=|y'|; (ii) removing spaces from x' and y' should get back x and y, respectively; and (iii) for any i, x'[i] and y'[i] cannot be both spaces.

A gap is a maximal substring of contiguous spaces in either x' or y'. An alignment A can include the following three kinds of regions: (i) matched pair (e.g., x'[i]=y'[i]; (ii) mismatched pair, (e.g., x'[i]≠y'[i] and both are not spaces); or (iii) gap (e.g., either x'[i ... j] or y'[i ... j] is a gap). In certain embodiments, only a matched pair has a high positive score a. In some embodiments, a mismatched pair generally has a negative score b and a gap of length r also has a negative score g+rs where g, s<0. For DNA, one common scoring scheme (e.g. used by BLAST) makes score a=1, score b=-3, g=-5 and s=-2. The score of the alignment A is the sum of the scores for all matched pairs, mismatched pairs and gaps. The alignment score of x and y can be defined as the maximum score among all possible alignments of x and y.

In some embodiments, any pair has a score a defined by a 4x4 matrix B of substitution probabilities. For example, B(i,i)=1 and 0<B(i,j)_{i<>j}<1 is one possible scoring system. For instance, where a transition is thought to be more biologically probable than a transversion, matrix B could include B(C,T)=0.7 and B(A,T)=0.3, or any other set of values desired or determined by methods known in the art.

Alignment according to some embodiments of the invention includes pairwise alignment. A pairwise alignment, generally, involves-for sequence Q (query) having m characters and a reference genome T (target) of n characters-finding and evaluating possible local alignments between Q and T. For any 1≦i≦n and 1≦j≦m, the largest possible alignment score of T[h ... i] and Q[k ... j], where h≦i and k≦j, is computed (i.e. the best alignment score of any substring of T ending at position i and any substring of Q ending at position j). This can include examining all substrings with cm characters, where c is a constant depending on a similarity model, and aligning each substring separately with Q. Each alignment is scored, and the alignment with the preferred score is accepted as the alignment. In some embodiments an exhaustive pairwise alignment is performed, which generally includes a pairwise alignment as described above, in which all possible local alignments (optionally subject to some limiting criteria) between Q and T are scored.

In some embodiments, pairwise alignment proceeds according to dot-matrix methods, dynamic programming methods, or word methods. Dynamic programming methods generally implement the Smith-Waterman (SW) algorithm or the Needleman-Wunsch (NW) algorithm. Alignment according to the NW algorithm generally scores aligned characters according to a similarity matrix S(a,b) (e.g., such as the aforementioned matrix B) with a linear gap penalty d. Matrix S(a,b) generally supplies substitution probabilities. The SW algorithm is similar to the NW algorithm, but any negative scoring matrix cells are set to zero. The SW and NW algorithms, and implementations thereof, are described in more detail in U.S. Pat. No. 5,701,256 and U.S. Pub. 2009/0119313, both herein incorporated by reference in their entirety. Computer programs known in the art for implementing these methods are described in more detail below.

In certain embodiments, an exhaustive pairwise alignment is avoided by positioning a consensus sequence or a contig along a reference genome through the use of a transformation of the sequence data. One useful category of transformation according to some embodiments of the invention involve making compressed indexes of sequences (see, e.g., Lam, et al., Compressed indexing and local alignment of DNA, 2008, Bioinformatics 24(6):791-97). Exemplary compressed indexes include the FN-index, the compressed suffix array, and the Burrows-Wheeler Transform (BWT, described in more detail below).

In certain embodiments, the invention provides methods of alignment which avoid an exhaustive pairwise alignment by making a suffix tree (sometime known as a suffix trie). Given a reference genome T, a suffix tree for T is a tree comprising all suffices of T such that each edge is uniquely labeled with a character, and the concatenation of the edge labels on a path from the root to a leaf corresponds to a unique suffix of T. Each leaf stores the starting location of the corresponding suffix.

On a suffix tree, distinct substrings of Tare represented by different paths from the root of the suffix tree. Then, Q is aligned against each path from the root up to cm characters (e.g., using dynamic programming). The common prefix structure of the paths also gives a way to share the common parts of the dynamic programming on different paths. A pre-order traversal of the suffix tree is performed; at each node, a dynamic programming table (DP table) is maintained for aligning the pattern and the path up to the node. More rows are added to the table while proceeding down the tree, and corresponding rows are deleted while ascending the tree.

In certain embodiments, a BWT is used to index reference T, and the index is used to emulate a suffix tree. The Burrows-Wheeler transform (BWT) (Burrow and Wheeler, 1994, A block-sorting lossless data compression algorithm, Technical Report 124, Digital Equipment Corporation, CA) was invented as a compression technique and later extended to support pattern matching. To perform a BWT, first let T be a string of length n over an alphabet E. Assume that the last character of T is a unique special character $, which is smaller than any character in E. The suffix array SA[0, n-1] of T is an array of indexes such that SA[i] stores the starting position of the i-th-lexicographically smallest suffix. The BWT of T is a permutation of T such that BWT [i]=T [SA[i]-1]. For example, if T='acaacg$', then SA=(8, 3, 1, 4, 2, 5, 6, 7), and BWT='gc$aaacc'.

Alignment generally involves finding the best alignment score among substrings of T and Q. Using a BWT of T speeds up this step by avoiding aligning substrings of T that are identical. This method exploits the common prefix structure of a tree to avoid aligning identical substrings more than once. Use of a pre-order traversal of the suffix tree generates all distinct substrings of T. Further, only substrings of T of length at most cm, where c is usually a constant bounded by 2, are considered, because the score of a match is usually smaller than the penalty due to a mismatch/insert/delete, and a substring of T with more than 2m characters has at most m matches and an alignment score less than 0. Implementation of the method for aligning sequence data is described in more detail in Lam, et al., Bioinformatics 24(6):791-97 (2008).

An alignment according to the invention can be performed using any suitable computer program known in the art.

One exemplary alignment program, which implements a BWT approach, is Burrows-Wheeler Aligner (BWA) available from the SourceForge web site maintained by Geeknet (Fairfax, Va.). BWA can align reads, contigs, or consensus sequences to a reference. BWT occupies 2 bits of memory per nucleotide, making it possible to index nucleotide sequences as long as 4G base pairs with a typical desktop or laptop computer. The pre-processing includes the construction of BWT (i.e., indexing the reference) and the supporting auxiliary data structures.

BWA implements two different algorithms, both based on BWT. Alignment by BWA can proceed using the algorithm bwa-short, designed for short queries up to ^{∼}200 bp with low error rate (<3%) (Li H. and Durbin R. Bioinformatics, 25:1754-60 (2009)). The second algorithm, BWA-SW, is designed for long reads with more errors (Li H. and Durbin R. (2010) Fast and accurate long-read alignment with Burrows-Wheeler Transform. Bioinformatics, Epub.). The BWA-SW component performs heuristic Smith-Waterman-like alignment to find high-scoring local hits. One skilled in the art will recognize that bwa-sw is sometimes referred to as "bwa-long", "bwa long algorithm", or similar. Such usage generally refers to BWA-SW.

An alignment program that implements a version of the Smith-Waterman algorithm is MUMmer, available from the SourceForge web site maintained by Geeknet (Fairfax, Va.). MUMmer is a system for rapidly aligning entire genomes, whether in complete or draft form (Kurtz, S., et al., Genome Biology, 5:R12 (2004); Delcher, A. L., et al., Nucl. Acids Res., 27:11 (1999)). For example, MUMmer 3.0 can find all 20-basepair or longer exact matches between a pair of 5-megabase genomes in 13.7 seconds, using 78 MB of memory, on a 2.4 GHz Linux desktop computer. MUMmer can also align incomplete genomes; it can easily handle the 100s or 1000s of contigs from a shotgun sequencing project, and will align them to another set of contigs or a genome using the NUCmer program included with the system. If the species are too divergent for a DNA sequence alignment to detect similarity, then the PROmer program can generate alignments based upon the six-frame translations of both input sequences.

Another exemplary alignment program according to embodiments of the invention is BLAT from Kent Informatics (Santa Cruz, Calif.) (Kent, W. J., Genome Research 4: 656-664 (2002)). BLAT (which is not BLAST) keeps an index of the reference genome in memory such as RAM. The index includes of all nonoverlapping k-mers (except optionally for those heavily involved in repeats), where k=11 by default. The genome itself is not kept in memory. The index is used to find areas of probable homology, which are then loaded into memory for a detailed alignment.

Another alignment program is SOAP2, from Beijing Genomics Institute (Beijing, CN) or BGI Americas Corporation (Cambridge, Mass.). SOAP2 implements a 2-way BWT (Li et al., Bioinformatics 25(15):1966-67 (2009); Li, et al., Bioinformatics 24(5):713-14 (2008)).

Another program for aligning sequences is Bowtie (Langmead, et al., Genome Biology, 10:R25 (2009)). Bowtie indexes reference genomes by making a BWT.

Other exemplary alignment programs include: Efficient Large-Scale Alignment of Nucleotide Databases (ELAND) or the ELANDv2 component of the Consensus Assessment of Sequence and Variation (CASAVA) software (Illumina, San Diego, Calif.); RTG Investigator from Real Time Genomics, Inc. (San Francisco, Calif.); Novoalign from Novocraft (Selangor, Malaysia); Exonerate, European Bioinformatics Institute (Hinxton, UK) (Slater, G., and Birney, E., BMC Bioinformatics 6:31(2005)), Clustal Omega, from University College Dublin (Dublin, Ireland) (Sievers F., et al., Mol Syst Biol 7, article 539 (2011)); ClustalW or ClustalX from University College Dublin (Dublin, Ireland) (Larkin M. A., et al., Bioinformatics, 23, 2947-2948 (2007)); and FASTA, European Bioinformatics Institute (Hinxton, UK) (Pearson W. R., et al., PNAS 85(8):2444-8 (1988); Lipman, D. J., Science 227(4693):1435-41 (1985)).

With each contig aligned to genomic sequences at genomic loci of at least one reference genome, the number of matching amplicons at individual loci can be counted. The number of amplicons matched to genomic loci on the chromosome(s) of interest can be compared to numbers of amplicons matched to genomic loci on the reference chromosome.

The output of the alignment includes an accurate and sensitive interpretation of the subject nucleic acid. The output can be provided in the format of a computer file. In certain embodiments, the output is a FASTA file, VCF file, text file, or an XML file containing sequence data such as a sequence of the nucleic acid aligned to a sequence of the reference genome. In other embodiments, the output contains coordinates or a string describing one or more mutations in the subject nucleic acid relative to the reference genome. Alignment strings known in the art include Simple UnGapped Alignment Report (SUGAR), Verbose Useful Labeled Gapped Alignment Report (VULGAR), and Compact Idiosyncratic Gapped Alignment Report (CIGAR) (Ning, Z., et al., Genome Research 11(10):1725-9 (2001)). These strings are implemented, for example, in the Exonerate sequence alignment software from the European Bioinformatics Institute (Hinxton, UK).

In some embodiments, the output is a sequence alignment-such as, for example, a sequence alignment map (SAM) or binary alignment map (BAM) file-comprising a CIGAR string (the SAM format is described, e.g., in Li, et al., The Sequence Alignment/Map format and SAMtools, Bioinformatics, 2009, 25(16):2078-9). In some embodiments, CIGAR displays or includes gapped alignments one-per-line. CIGAR is a compressed pairwise alignment format reported as a CIGAR string. A CIGAR string is useful for representing long (e.g. genomic) pairwise alignments. A CIGAR string is used in SAM format to represent alignments of reads to a reference genome sequence.

A CIGAR string follows an established motif. Each character is preceded by a number, giving the base counts of the event. Characters used can include M, I, D, N, and S (M=match; I=insertion; D=deletion; N=gap; S=substitution). The cigar line defines the sequence of matches/mismatches and deletions (or gaps). For example, the cigar line 2MD3M2D2M will mean that the alignment contains 2 matches, 1 deletion (number 1 is omitted in order to save some space), 3 matches, 2 deletions and 2 matches.

To illustrate, if the original sequence is AACGCTT and the CIGAR string is 2MD3M2D2M, the aligned sequence will be AA-CGG-TT. As a further example, if an 80 bp read aligns to a contig such that the first 5' nucleotide of the read aligns to the 50th nucleotide from the 5' end of the contig with no indels or substitutions between the read and the contig, the alignment will yield "80M" as a CIGAR string.

In certain embodiments, as part of the analysis and determination of copy number states and subsequent identification of copy number variation, the sequence read counts for genomic regions of interest can be normalized based on internal controls. In particular, an intra-sample normalization is performed to control for variable sequencing depths between samples. The sequence read counts for each genomic region of interest within a sample will be normalized according to the total read count across all control references within the sample.

After normalizing read counts for both the genomic regions of interest and control references, copy number states can be determined. In one embodiment, the normalized values for each sample of interest will be compared to the normalized values for a control sample. A ratio, for example, may be generated based on the comparison, wherein the ratio is indicative of copy number and further determinative of any copy number variation. In the event that the determined copy number of a genomic region of interest of a particular sample falls within a tolerable level (as determined by ratio between test and control samples), thus indicating that there are two copies of the chromosome containing the region of interest. In the event that the determined copy number of a genomic region of interest of a particular sample falls outside of a tolerable level, it can be determined that genomic region of interest does present copy number variation and thus the cells are aneuploidy.

For example, based on the ratios, loci copy numbers can be called as follows: a ratio of <0.1 can be called a copy number state of 0; a ratio between 0.1 and 0.8 can be called a copy number state of 1 (monosomy); a ratio between 0.8 and 1.25 can be called a copy number state of 2 (disomy); and a ratio of >1.25 can be called a copy number state of 3+ (e.g, trisomy).

The determined copy numbers can then be used to determine a euploidy or aneuploidy state of the embryo . In particular, if the copy number state is determined to vary from the normal copy state (e.g., CN is 0, 1 or 3+), it is indicative of aneuploidy.

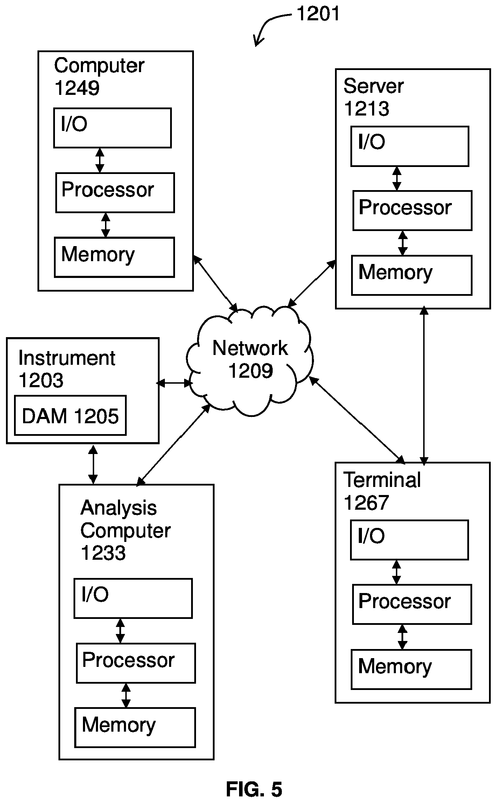

As one skilled in the art would recognize as necessary or best-suited for performance of the methods of the invention and sequence assembly in general, a computer system(s) or machine(s) can be used. FIG. 5 gives a diagram of a system 1201 according to embodiments of the invention. System 1201 may include an analysis instrument 1203 which may be, for example, a sequencing instrument (e.g., a HiSeq 2500 or a MiSeq by Illumina). Instrument 1203 includes a data acquisition module 1205 to obtain results data such as sequence read data. Instrument 1203 may optionally include or be operably coupled to its own, e.g., dedicated, analysis computer 1233 (including an input/output mechanism, one or more processor, and memory). Additionally or alternatively, instrument 1203 may be operably coupled to a server 1213 or computer 1249 (e.g., laptop, desktop, or tablet) via a network 1209.

Computer 1249 includes one or more processors and memory as well as an input/output mechanism. Where methods of the invention employ a client/server architecture, steps of methods of the invention may be performed using the server 1213, which includes one or more of processors and memory, capable of obtaining data, instructions, etc., or providing results via an interface module or providing results as a file. The server 1213 may be engaged over the network 1209 by the computer 1249 or the terminal 1267, or the server 1213 may be directly connected to the terminal 1267, which can include one or more processors and memory, as well as an input/output mechanism.

In system 1201, each computer preferably includes at least one processor coupled to a memory and at least one input/output (I/O) mechanism.

A processor will generally include a chip, such as a single core or multi-core chip, to provide a central processing unit (CPU). A process may be provided by a chip from Intel or AMD.

Memory can include one or more machine-readable devices on which is stored one or more sets of instructions (e.g., software) which, when executed by the processor(s) of any one of the disclosed computers can accomplish some or all of the methodologies or functions described herein. The software may also reside, completely or at least partially, within the main memory and/or within the processor during execution thereof by the computer system. Preferably, each computer includes a non-transitory memory such as a solid state drive, flash drive, disk drive, hard drive, etc. While the machine-readable devices can in an exemplary embodiment be a single medium, the term "machine-readable device" should be taken to include a single medium or multiple media (e.g., a centralized or distributed database, and/or associated caches and servers) that store the one or more sets of instructions and/or data. These terms shall also be taken to include any medium or media that are capable of storing, encoding, or holding a set of instructions for execution by the machine and that cause the machine to perform any one or more of the methodologies of the present invention. These terms shall accordingly be taken to include, but not be limited to one or more solid-state memories (e.g., subscriber identity module (SIM) card, secure digital card (SD card), micro SD card, or solid-state drive (SSD)), optical and magnetic media, and/or any other tangible storage medium or media.

A computer of the invention will generally include one or more I/O device such as, for example, one or more of a video display unit (e.g., a liquid crystal display (LCD) or a cathode ray tube (CRT)), an alphanumeric input device (e.g., a keyboard), a cursor control device (e.g., a mouse), a disk drive unit, a signal generation device (e.g., a speaker), a touchscreen, an accelerometer, a microphone, a cellular radio frequency antenna, and a network interface device, which can be, for example, a network interface card (NIC), Wi-Fi card, or cellular modem.

Other embodiments are within the scope and spirit of the invention. For example, due to the nature of software, functions described above can be implemented using software, hardware, firmware, hardwiring, or combinations of any of these. Features implementing functions can also be physically located at various positions, including being distributed such that portions of functions are implemented at different physical locations.

Aneuploidy status of a sample can also be determined by comparison of z-scores. This is done by first determining the mean and standard deviation of tag counts within a chromosome of interest in a group of reference samples, wherein the references samples have known euploid content. Then, a standardized score (i.e., z-score) is created for each chromosome of interest for each sample using the following equation: z-score_{i,chrN}=(chrNᵢ-µ_{chrN}) sd_{chrN}, where i represents the sample to be standardized, chrN represents the normalized tag count of the sample's chromosome, and µ_{chrN} and sd_{chrN} represent the mean and standard deviation of the normalized tag counts, respectively, of chrN in the reference group. Typically, a z-score greater 3 identifies an outlier and indicates that the normalized tag count of the outlier exceeds the mean of the reference group by at least three standard deviations. However, a z-score lower than three, such as, for example, 2, can also identify an outlier.

### INCORPORATION BY REFERENCE

References and citations to other documents, such as patents, patent applications, patent publications, journals, books, papers, web contents, have been made throughout this disclosure. All such documents are hereby incorporated herein by reference in their entirety for all purposes.

### EQUIVALENTS

Various modifications of the invention and many further embodiments thereof, in addition to those shown and described herein, will become apparent to those skilled in the art from the full contents of this document, including references to the scientific and patent literature cited herein. The subject matter herein contains important information, exemplifications and guidance that can be adapted to the practice of this invention in its various embodiments and equivalents thereof.

### EXAMPLES

### Example 1

153 samples of 12 pg purified genomic DNA were obtained from 19 aneupoid cell lines. DNA was derived from transformed lymphocytes at the equivalent of 2 cells/reaction. In accordance with the methods according to the methods shown in FIGs 2 and 3, nucleic acid was obtained from the samples, subject to PCR reactions, and the products were sequenced to generate count data for each chromosome, the count data being subsequently used to infer karyotypes.

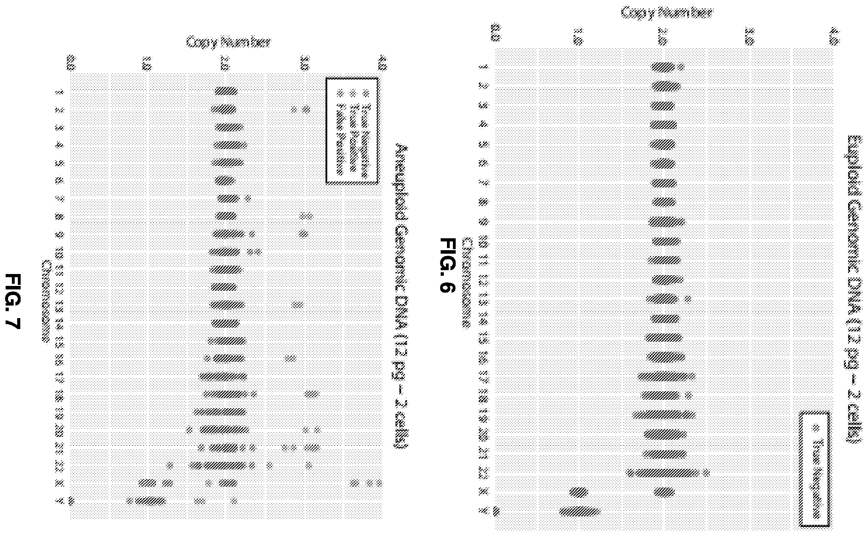

FIG. 6 shows the results from euploid cells and FIG. 7 shows the results from the aneuploidy cells. A total of 41 true aneuploid chromosome calls, 3630 true diploid chromosome calls, 1 incorrect aneuploid (false positive) chromosome call, and 0 incorrect diploid (false negative) chromosome calls were made. The incorrect aneuploid call was in a sample that contains other aneuploid chromosomes, thus yielding perfect sample-level specificity, and perfect sample- and chromosome-level sensitivity.

Aneuploidies detected included trisomies 2, 8, 9, 13, 18, 20, 21, 22, 2+21, and 16+21, XO, XXXX, XXY, and XYY.

### Example 2

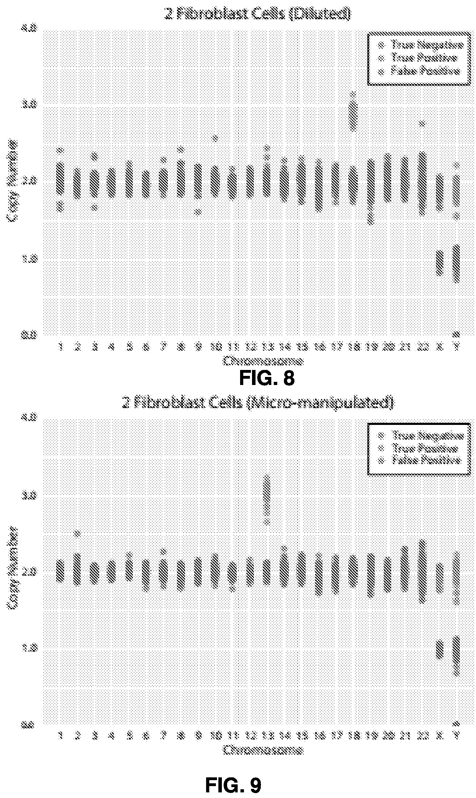

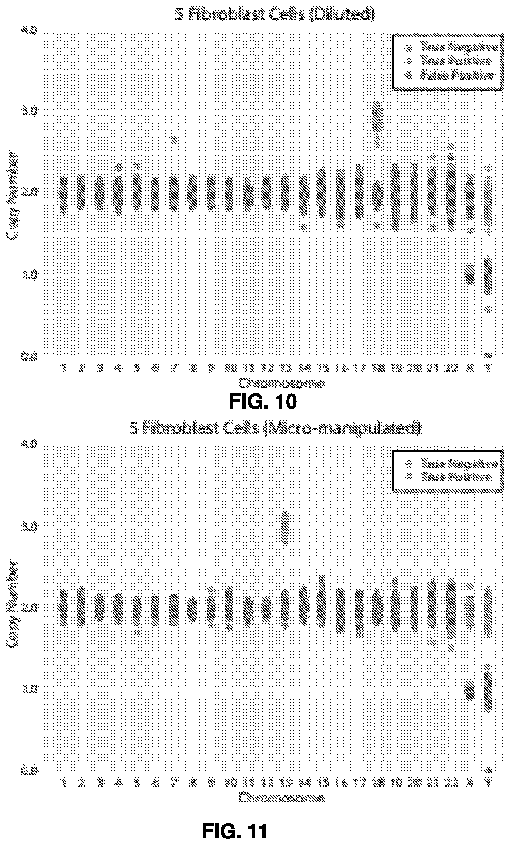

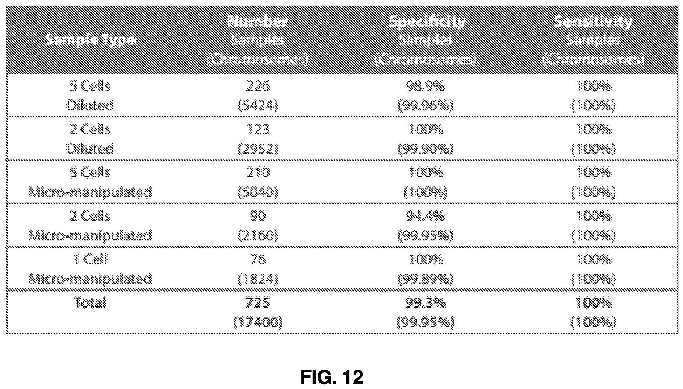

Lysate was derived from 1 to 5 cultured fibroblast cells. In accordance with the methods according to the methods shown in FIGs 2 and 3, nucleic acid was obtained from the samples, subject to PCR reactions, and the products were sequenced to generate count data for each chromosome, the count data being subsequently used to infer karyotypes. The aneuploidies detected were trisomy 13, trisomy 18, XXY, and XYY when lysate from one, two, or five fibroblasts was used as template. The results can be seen in FIGs. 8-11. FIGs 8 and 9 show the karyotype calls when only two fibroblast cells were used. The cells in FIG. 8 were diluted, while the cells in FIG. 9 were micro-manipulated. FIGs. 10 and 11 show the karyotype calls when five fibroblast cells were used. The cells in FIG. 10 were diluted, while the cells in FIG. 11 were micro-manipulated. FIG. 12 summarizes the number, specificity and sensitivity by number of fibroblast cells and whether they were diluted or micro-manipulated. As can be seen from the table, close to 100% specificity was reached with both diluted and micro-manipulated samples across samples from 1-5 cells and 100% sensitivity was reached with all samples types.

Embodiments of the invention may include the features of the following enumerated paragraphs ("paras").
1. A method for determining ploidy of an embryo, the method comprising:
   amplifying, using a primer pair that amplifies a plurality of human genomic loci, nucleic acid from a preimplantation embryo to generate a plurality of amplicons;
   sequencing the amplicons to generate a plurality of sequence reads;
   matching the sequence reads to the genomic loci and counting a number of matches; and
   determining chromosome count based on the number of matches.
2. The method of para 1, further comprising obtaining a sample of nucleic acid.
3. The method of para 2, further comprising obtaining the sample by biopsy.
4. The method of para 3, wherein the biopsy is a trophectoderm biopsy.
5. The method of para 2, wherein the sample includes at least one cell from the preimplantation embryo.
6. The method of para 5, wherein the sample contains from about 1 to about 8 cells.
7. The method of para 6, wherein the sample contains from about 1 to about 5 cells.
8. The method of para 1, wherein the primer pair is complimentary to sequences distributed on at least 4 human chromosomes.
9. The method of para 1, wherein not all of the amplicons are identical.
10. The method of para 1, wherein the amplicons include sequences on at least one chromosome of interest and sequences on one or more reference chromosomes.
11. The method of para 10, wherein the at least one chromosome of interest is selected from the group consisting of chromosome 9, chromosome 13, chromosome 18, chromosome 21, X chromosome and Y chromosome.
12. The method of para 1, wherein the determining chromosome count step comprises the generation and comparison of a z-score for a chromosome of interest.
13. The method of para 1, further comprising determining a euploidy or aneuploidy state of the embryo based on the chromosome count.
14. The method of para 1, further comprising attaching sequence adapters and bar codes to the amplicons simultaneously with amplification of the nucleic acid.
15. The method of para 1, wherein the primer comprises a universal primer binding site.
16. The method of para 15, further comprising a second round of amplification comprising adding sequencing adaptors to the amplicons using second primers that hybridize to the universal primer binding site.
17. The method of para 1, further comprising fragmenting the nucleic acid.
18. A system for determining chromosome count, the system comprising:
   a processor coupled to a tangible memory subsystem storing instructions that when executed by the processor cause the system to:
   obtain sequence reads from amplicons, wherein the amplicons are generated by amplifying, using a primer pair that amplifies a plurality of human genomic loci, nucleic acid from a preimplantation embryo;
   match the sequence reads to the genomic loci;
   count a number of matches at the genomic loci; and
   determine chromosome count based on the number of matches.
19. The system of para 18, wherein the nucleic acid was obtained from a sample.
20. The system of para 19, wherein the sample was obtained by biopsy
21. The system of para 20, wherein the biopsy is a trophectoderm biopsy.
22. The system of para 19, wherein the sample contains from about 1 to about 5 cells from the preimplantation embryo.
23. The system of para 19, wherein the primer pair is complimentary to sequences distributed on at least 4 human chromosomes.
24. The system of para 19, wherein the amplicons include sequences on at least one chromosome of interest and sequences on one or more reference chromosomes.
25. The system of para 24, wherein the at least one chromosome of interest is selected from the group consisting of chromosome 9, chromosome 13, chromosome 18, chromosome 21, X chromosome and Y chromosome.
26. The system of para 1, wherein the instructions further cause the system to determine and report a euploidy or aneuploidy state of the embryo based on the chromosome count.

## Claims

1. A method for determining ploidy of an embryo, the method comprising:
amplifying nucleic acid from an isolated preimplantation embryo sample to generate a plurality of amplicons;
sequencing the plurality of amplicons to generate a plurality of sequence reads;
matching the plurality of sequence reads to a plurality of human genomic loci and counting a number of matches to determine a plurality of read counts for each genomic locus; and
determining a chromosome count for the embryo based on the number of matches.

2. The method of claim 1, further comprising:
normalizing the plurality of read counts for each genomic locus according to a total read count of all control references in the sample; and
generating a ratio based on the normalized plurality of read counts for each genomic locus and the total read count of all control references in the sample,
wherein determining the chromosome count comprises determining the chromosome count for the embryo based on the ratio.

3. The method of claim 1, further comprising:
obtaining, by a trophectoderm biopsy, the isolated preimplantation embryo sample from a preimplantation embryo; and
vitrifying the preimplantation embryo.

4. The method of claim 1, further comprising lysing the isolated preimplantation embryo sample to release the nucleic acid.

5. The method of claim 1, further comprising fragmenting the nucleic acid to generate a plurality of fragments, wherein amplifying the nucleic acid comprises amplifying the plurality of fragments to generate the plurality of amplicons.

6. The method of claim 1, wherein amplifying the nucleic acid from the isolated preimplantation embryo sample to generate a plurality of amplicons comprises using a primer pair that amplifies the plurality of human genomic loci.

7. The method of claim 1, wherein not all of the plurality of amplicons are identical or the plurality of amplicons include sequences on at least one chromosome of interest and sequences on one or more reference chromosomes; and in which case optionally wherein the at least one chromosome of interest is selected from the group consisting of chromosome 9, chromosome 13, chromosome 18, chromosome 21, X chromosome, and Y chromosome.

8. The method of claim 1, wherein determining the chromosome count comprises generating and comparing a z-score for a chromosome of interest.

9. The method of claim 1, further comprising determining a euploidy or aneuploidy state of the embryo based on the chromosome count.

10. A system for determining ploidy of an embryo, the system comprising:
a memory; and
a processor coupled to the memory, the processor configured to:
amplify nucleic acid from an isolated preimplantation embryo sample to generate a plurality of amplicons;
sequence the plurality of amplicons to generate a plurality of sequence reads;
match the plurality of sequence reads to a plurality of human genomic loci and counting a number of matches to determine a plurality of read counts for each genomic locus; and
determine a chromosome count for the embryo based on the number of matches.

11. The system of claim 10, wherein the processor is further configured to:
normalize the plurality of read counts for each genomic locus according to a total read count of all control references in the sample; and
generate a ratio based on the normalized plurality of read counts for each genomic locus and the total read count of all control references in the sample,
wherein determining the chromosome count comprises determining the chromosome count for the embryo based on the ratio.

12. The system of claim 10, wherein the preimplantation embryo sample is obtained by a trophectoderm biopsy of a preimplantation embryo and the preimplantation embryo is vitrified.

13. The system of claim 10, wherein the isolated preimplantation embryo sample is lysed to release the nucleic acid.

14. The system of claim 10, wherein the nucleic acid is fragmented acid to generate a plurality of fragments, and wherein the amplification of the nucleic acid comprises amplifying the plurality of fragments to generate the plurality of amplicons.

15. A non-transitory computer-readable medium having instructions stored thereon, execution of which, by one or more processors of a device, cause the one or more processors to perform operations comprising:
amplifying nucleic acid from an isolated preimplantation embryo sample to generate a plurality of amplicons;
sequencing the plurality of amplicons to generate a plurality of sequence reads;
matching the plurality of sequence reads to a plurality of human genomic loci and counting a number of matches to determine a plurality of read counts for each genomic locus; and
determining a chromosome count for the embryo based on the number of matches.
